(19) Europäisches Patentamt
European Patent Office
Office européen des brevets

(11) Numéro de publication: **0 476 120 B1**

(12) **FASCICULE DE BREVET EUROPEEN**

(45) Date de publication de fascicule du brevet: **28.06.95**

(51) Int. Cl.[6]: **C07C 229/24**, A61K 31/195, A61K 31/55

(21) Numéro de dépôt: **91907714.9**

(22) Date de dépôt: **05.04.91**

(86) Numéro de dépôt internationale :
**PCT/FR91/00284**

(87) Numéro de publication internationale :
**WO 91/15463 (17.10.91 91/24)**

# (54) PROCEDE DE PREPARATION D'UN SEL EOUIMOLAIRE D'ACIDE ASPARTIOUE ET DE MAGNESIUM, SEL D'ASPARTATE DE MAGNESIUM OBTENU PAR LEDIT PROCEDE ET SES APPLICATIONS.

(30) Priorité: **05.04.90 FR 9004353**

(43) Date de publication de la demande:
**25.03.92 Bulletin 92/13**

(45) Mention de la délivrance du brevet:
**28.06.95 Bulletin 95/26**

(84) Etats contractants désignés:
**AT BE CH DE DK ES FR GB GR IT LI LU NL SE**

(56) Documents cités:
**EP-A- 0 108 937**
**DE-C- 357 754**
**GB-A- 910 321**
**GB-A- 955 087**
**GB-A- 1 283 810**
**US-A- 3 836 668**
**US-A- 4 137 326**

**THE BIOCHEMICAL JOURNAL, vol. 21, 1927, pages 1168-1178, W. K. ANSLOW et al: "CLVI. Neutral salt addition compounds of alkaline earth glutamates and aspartates"**

(73) Titulaire: **LABORATOIRES MAYOLY SPINDLER**
**6, avenue de l'Europe,**
**B.P. 51**
**F-78401 Chatou Cédex (FR)**

(72) Inventeur: **CHARAGNAC, Jean-Luc**
**14, rue de Vauboyen**
**F-91570 Bièvres (FR)**

(74) Mandataire: **Ores, Irène et al**
**CABINET ORES**
**6, Avenue de Messine**
**F-75008 Paris (FR)**

**EP 0 476 120 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

**JOURNAL OF INORGANIC AND NUCLEAR CHEMISTRY, vol. 35, no. 7, July 1973, pages 2441-2446, Oxford, GB; R. C. TEWARI et al: "Formation and stabilities of some bivalent metal ion chelates of 1-glutamine"**

**JOURNAL OF THE ELECTROCHEMICAL SOCIETY OF INDIA, vol. 30, no. 4, 1981, pages 336-340, Bangalore; R. RAMAJJE GOWDA et al: "Interaction of acidic amino acids with bivalent metal ions"**

## Description

La présente invention est relative à un procédé de préparation de sels équimolaires d'un acide α-aminé dicarboxylique et d'un métal divalent, à un sel équimolaire d'acide aspartique et de magnésium obtenu par ledit procédé, et à des médicaments comprenant ledit sel.

Les acides α-aminés dicarboxyliques, répondent à la formule générale qui est représentée ci-dessous :

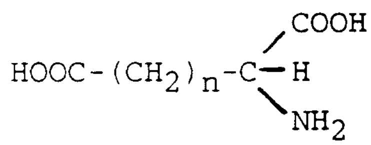

A titre d'exemples d'acides α-aminés dicarboxyliques, on citera deux acides α-aminés naturels, l'acide aspartique et l'acide glutamique.

Les acides α-aminés dicarboxyliques possèdent deux fonctions carboxyliques ionisables ; il semble donc théoriquement possible d'obtenir un sel équimolaire d'un métal divalent quelconque et d'un acide α-aminé dicarboxylique.

Le Brevet Anglais 910 321 décrit l'obtention d'un sel équimolaire d'acide aspartique et de fer. Le Brevet Anglais 955 087 cite un sel équimolaire d'acide glutamique et de magnésium, en tant qu'intermédiaire pour la fabrication de bromhydrate de magnésium.

Toutefois, en ce qui concerne les sels d'acide aspartique et de magnésium, il n'avait jusqu'à présent été possible que d'obtenir des hémisels dans lesquels le rapport molaire magnésium/acide aspartique est égal à 1/2.

Ceci est probablement dû à l'arrangement octaédrique très privilégié représenté ci-dessous.

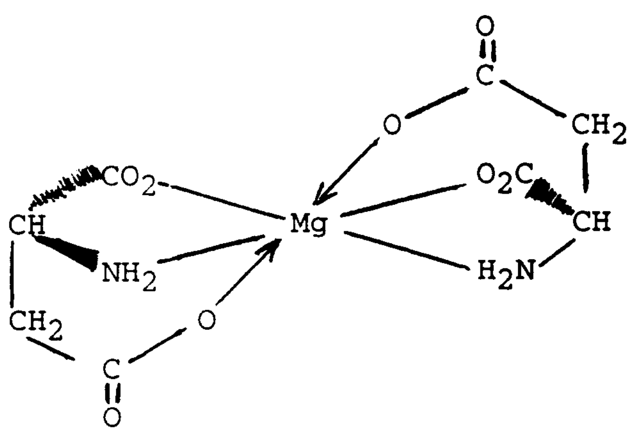

Des recherches ont été faites dans le but d'augmenter le rapport magnésium/acide aspartique, dans le but de se rapprocher du rapport théorique 1/1.

On trouve ainsi dans l'art antérieur une méthode permettant d'obtenir un sel d'aspartate "enrichi en magnésium" ( Brevet Allemand 3 238 118, au nom de VERLA PHARM.).

Ce Brevet décrit la préparation en une étape, à partir d'aspartate d'un métalloxyde (en l'occurrence MgO) et d'un métalhalogène (le chlorure de magnésium), d'un chlorhydrate d'aspartate de magnésium.

La structure tridimensionnelle avancée pour ce sel est représentée ci-dessous :

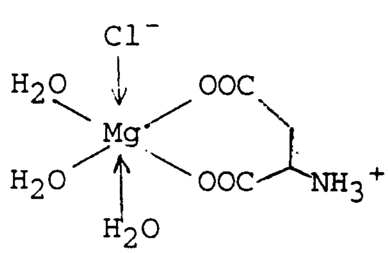

La présente invention s'est fixé pour but la mise au point d'un procédé d'obtention de sels d'acide α-aminé dicarboxylique et de métal divalent, qui, contrairement aux procédés connus dans l'art antérieur, permet l'obtention de sels non halogénés d'aspartate de magnésium, associant ces deux constituants en proportions équimolaires, ce qui permet d'obtenir des sels plus concentrés en magnésium que ceux connus dans l'art antérieur.

La présente Invention a pour objet un sel d'acide aspartique et de magnésium, caractérisé en ce qu'il répond à la formule brute suivante :

$C_4 H_5 O_4 NMg, 2H_2O$.

La présente Invention a également pour objet un procédé de préparation d'un sel équimolaire d'acide aspartique et de magnésium, lequel procédé est caractérisé en ce qu'il comprend une étape dans laquelle on procède à la déprotonnation de l'acide aspartique en milieu hétérogène, en présence d'hydroxyde de magnésium.

Selon un mode de mise en oeuvre préféré du procédé conforme à l'invention, l'hydroxyde de magnésium est en excès molaire par rapport à l'acide aspartique.

Le procédé conforme à l'invention utilise un protocole simple, et le produit obtenu ne contient, comme contaminant éventuel, qu'un excès d'hydroxyde, qui est facilement éliminé, par exemple par filtration.

Le sel d'aspartate obtenu peut être précipité par l'addition d'acétone, mais il est également possible de supprimer cette dernière étape en séchant la solution par atomisation, après élimination de l'excès d'hydroxyde. Il apparaîtra clairement à l'homme de l'art que d'autres procédés de séchage, par exemple la lyophilisation, peuvent également être utilisés.

La présente Invention a en outre pour objet des compositions pharmaceutiques caractérisées en ce qu'elles contiennent un sel équimolaire d'acide aspartique et de magnésium conforme à l'Invention, seul ou en association avec un autre principe actif.

Les hémisels d'acide α-aminé dicarboxylique (en particulier d'acide glutamique et d'acide aspartique) et de métal divalent connus dans l'art antérieur sont utilisés dans le traitement des états d'asthénie fonctionnelle. Ils sont considérés comme constituant une forme qui favorise particulièrement l'absorption par l'organisme des acides α-aminés dicarboxyliques aussi bien que des métaux divalents qui y sont associés .

Le sel équimolaire d'acide aspartique et de magnésium conforme à l'Invention est utilisable dans les mêmes indications que les hémisels; il possède en outre sur ces derniers l'avantage de permettre, à quantité égale de sel, l'administration d'une plus grande proportion de métal divalent.

Les Inventeurs ont également constaté que le sel équimolaire d'aspartate et de magnésium obtenu conformément à l'Invention possédait un effet anxiolytique léger.

Le terme "anxiolytique" se rapporte, en pharmacologie, à une classe de médicaments ayant comme point commun leur utilisation thérapeutique pour réduire l'anxiété ; en général, les anxiolytiques ont un effet sédatif à faible dose, et hypnotique à dose plus élevée ; certains d'entre eux ont en outre un effet myorelaxant. Parmi les substances couramment utilisées comme anxiolytiques, on citera, à titre indicatif, les benzodiazépines, les carbamates, le clométiazole, les dérivés du diphénylméthane.

Les Inventeurs ont en outre mis en évidence une synergie d'action inattendue entre le sel d'acide aspartique et de magnésium conforme à l'Invention, et des anxiolytiques tels que les benzodiazépines ; cette synergie se manifeste par une potentialisation importante autant que surprenante des effets, en particulier sédatifs, des anxiolytiques considérés lorsqu'ils sont associés au sel conforme à l'invention. Les compositions résultant de cette association permettent donc de réduire considérablement, pour un effet équivalent, la dose d'anxiolytique administrée.

Selon un mode de réalisation préféré des compositions pharmaceutiques conformes à l'invention, elles contiennent, en tant que principe actif, un sel équimolaire d'acide aspartique et de magnésium en association avec un anxiolytique.

Selon encore une disposition préférée de ce mode de réalisation, l'anxiolytique est une benzodiazépine.

La présente Invention sera mieux comprise à l'aide du complément de description qui va suivre, qui se réfère à des exemples de mise en oeuvre du procédé d'obtention d'un sel équimolaire d'acide aspartique et de magnésium conforme à l'invention, et à la démonstration de l'activité pharmacologique du sel équimolaire d'aspartate de magnésium obtenu par ledit procédé.

Il doit être bien entendu, toutefois, que ces exemples de préparation, tests et études pharmacologiques sont donnés uniquement à titre d'illustration de l'objet de l'invention, dont ils ne constituent en aucune manière une limitation.

## I) PREPARATION DE SELS EOUIMOLAIRES D'ACIDES $\alpha$-AMINES DICARBOXYLIOUES ET DE METAUX DIVALENTS CONFORMES A L'INVENTION

### Exemple 1 - Préparation d'un sel équimolaire de magnésium et d'acide aspartique

A/ - PROTOCOLE EXPERIMENTAL

L'acide aspartique (10g) et l'hydroxyde de magnésium (6g) sont mis en suspension dans 50 ml d'eau distillée, puis, sous agitation magnétique, le mélange est chauffé à 90°C (température du bain) pendant 24 heures.

La solubilité de l'hydroxyde de magnésium dans l'eau étant très faible, on a utilisé ce réactif en excès et chauffé ce mélange pour améliorer le rendement de la réaction.

Néanmoins, la très grande solubilité de l'aspartate de magnésium obtenu a pour effet de déplacer l'équilibre de la réaction dans le sens de la production de ce sel.

Après refroidissement du mélange, l'excès d'hydroxyde est éliminé par filtration sur papier WATMAN n° 5. Le sel d'acide aspartique et de magnésium est précipité en additionnant au goutte à goutte un volume égal d'acétone (environ 50 ml).

Par maintien à 4°C pendant 2 heures, le précipité forme une pâte. On élimine le surnageant, puis on rince avec un peu d'acétone. Après redissolution, dans une quantité minimale d'eau, l'on procède à la lyophilisation, à l'issue de laquelle on récupère une poudre blanche très légèrement jaune.

Ce produit est hygroscopique et doit être stocké à l'abri de l'humidité.

20g du produit obtenu sont solubles dans 100 ml d'eau à 20 °C . En solution, le composé est stable à température ambiante, le pH est de 9,8.

Une solution à 2% dans l'acide chlorhydrique 2N possède un pouvoir rotatoire de + 17°,9 ce qui indique que le produit n'est pas un mélange racémique.

B/ - COMPOSITION ET STRUCTURE DU COMPOSE OBTENU

. Microanalyse : sur $C_4H_5NMg.2H_2O$

| | Pourcentages théoriques | Pourcentages observés |
|---|---|---|
| C | 25,1 | 25,85 |
| H | 4,74 | 4,94 |
| N | 7,31 | 7,2 |

Ce composé est hygroscopique et s'hydrate rapidement à l'air. Cette propriété peut expliquer la légère différence entre les pourcentages observés et les pourcentages théoriques. Un dosage fiable de l'oxygène est impossible en présence de magnésium. La microanalyse indique que la réaction est complète et qu'on n'isole pas d'acide aspartique sous sa forme de départ.

Dosage du magnésium: Il est réalisé par complexométrie : Pourcentage théorique : 12,69% (p/p) ; pourcentage observé : 11,8%(p/p).

(A titre de comparaison le pourcentage en poids de magnésium des hémisels de magnésium et d'acide aspartique connus dans l'art antérieur est de 6,6%)

. RMN du proton (200 $MH_2$,$D_2O$) - (Figure 1)

On observe 2 massifs à 2,4 ppm et 3,4 ppm dont l'intensité correspond respectivement à 2 protons et 1 proton.

Attribution :

2,4

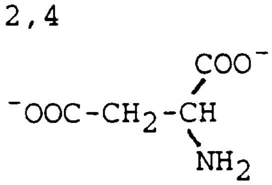

3,4

Les protons de l'amine sont échangeables et n'apparaissent pas. La proximité du centre asymétrique rend théoriquement différents les 2 protons de méthylène : on a donc réalisé une RMN du proton à haut champ (400 MHz, $D_2O$) (Figure 2) afin d'obtenir une meilleure résolution des massifs.

Le massif à 2,4 ppm est alors effectivement résolu en une paire de double de doublet, révélant un couplage Gem.J = 18Hz entre les 2 protons du méthylène et une constante de couplage avec le proton du carbone adjacent (J = 2Hz et J = 5Hz).

Le signal à 3,4 ppm, superposition des petites constantes de couplage J = 2Hz et J = 5Hz n'est pas convenablement résolu.

La structure microcristalline du produit obtenu ne permet pas l'obtention de données cristallographiques par les rayons X. Sur la base de la teneur en magnésium, des résultats de la RMN du proton et de la microanalyse, il est toutefois possible de déterminer la formule brute du sel obtenu, qui est :

$C_4\ H_5\ O_4\ N\ Mg,\ 2\ H_2O$

ce qui correspond bien à un sel équimolaire d'aspartate et de magnésium dihydraté ASPMg,$2H_2O$

## II) DEMONSTRATION DE L'ACTIVITE PHARMACOLOGIOUE DES COMPOSES CONFORMES A L'INVENTION

A). - EFFET ANXIOLYTIQUE DE L'$\alpha$-$\beta$-ASPARTATE DE MAGNESIUM

**EXEMPLE 2: Effet de l'$\alpha$-$\beta$-aspartate de magnésium en prise unique sur le comportement exploratoire en enceinte non anxiogène et anxiogène, et la coordination motrice chez la souris.**

Etude du comportement exploratoire:

Le principe de cette étude est basé sur le fait qu'une souris placée pour la première fois dans un environnement nouveau manifeste une activité motrice très vive afin d'explorer ce lieu, quel qu'il soit ; l'étude cinétique de l'activité motrice montre que cette dernière est maximum dans les 5 premières minutes puis décroît de façon notable de telle sorte qu'entre 5 et 15 minutes (c'est-à-dire en 10 minutes) les scores sont égaux à ceux notés dans les 5 premières minutes.

Les scores d'activité sont fonction de la surface à explorer. Ils sont également fonction de deux motivations contradictoires : la curiosité et la crainte. Il faut enfin savoir que lorsque la surface offerte permet une activité "horizontale" (marche) la curiosité parait l'emporter ; par contre lorsque la surface est réduite et conduit à une activité "verticale" (redressements le long des parois d'un cylindre) il semble que la crainte anxieuse soit davantage présente et conduise à une activité soit diminuée, soit cyclique, et apparemment sans focalisation.

Cet état d'anxiété est confirmé par la présence d'une défécation et d'une miction que nous qualifions d'émotionnelles.

Etude de la coordination motrice:

Le test dit du "rota-rod" permet de juger le tonus musculaire et la coordination motrice de souris placées sur une tige tournant à vitesse constante.

1) PROTOCOLE EXPERIMENTAL

Ces études ont été réalisées chez des souris mâles, de souche NMRI-Han, réparties au hasard en lots de 15 souris (séries traitées) et 20 souris (série contrôle).

1.1) Motilité spontanée

La motilité a été mesurée à l'aide d'un actimètre "type Dews" (Brit. J. Pharmacol. 1953, 8 : 46). Les animaux sont placés un par un dans une enceinte rectangulaire en matière plastique transparente (mesures intérieures : L = 24 cm, l = 15 cm, h = 9,5 cm), couverte par un grillage métallique et traversée par deux faisceaux infra-rouges perpendiculaires situés à 2 cm au-dessus du plancher (médianes de l'enceinte) ; ces deux faisceaux viennent frapper deux cellules photo-électriques reliées à un compteur d'impulsions déclenché à chaque fois que l'animal coupe un des faisceaux. Les actimètres ont été placés dans une

pièce non insonorisée mais en dehors de tout bruit aigu, à une température de l'ordre de 21 °C.

L'activité motrice a été mesurée au cours des 5 premières minutes (exploration non anxiogène) puis entre 5 et 15 minutes (activité basale non anxiogène).

1.2) Motilité dans des conditions anxiogènes

Au sortir de l'actimètre, les souris sont placées dans un cylindre en verre (hauteur 30 cm, diamètre 9 cm)

L'exploration anxiogène a été mesurée par le nombre de redressements au cours de la première minute passée dans le cylindre, l'activité basale anxiogène étant considérée dans la même enceinte, entre 1 et 3 minutes.

Enfin, l'effet à l'égard des manifestations dites émotionnelles est mesuré sur la miction et la défécation au cours des 3 minutes passées dans le cylindre et permet le calcul d'une DA 50 dite "anti-émotionnelle".

1.3) Effet sur le tonus musculaire

La DA 50 s'exprime par la dose qui provoque en moins de 3 minutes la chute de 50 p. 100 des souris placées sur une tige en bois tournant à une vitesse de 16 tours/min (diamètre 1,7 cm). Ce test qui permet de juger de la coordination motrice des souris est adapté de la technique décrite par N.W. DUNHAN and T.S. MIYA (J. Am. Pharm. Assoc. 1957, 46 : 208)

1.4) Répartition des souris et posologie

Quatre-vingt souris ont été réparties de la façon suivante :

| Séries d'animaux | Doses $mg.kg^{-1}$ P.O | Nombre |
|---|---|---|
| Contrôle | 0 | 20 |
| $\alpha$-$\beta$-aspartate de magnésium | 175<br>350<br>700<br>1400 | 15<br>15<br>15<br>15 |

Les doses ont été calculées selon une progression géométrique de base 700 (correspondant au 1/10 de la DL 50) et de raison 2, soit respectivement 175, 350, 700 et 1400 $mg.kg^{-1}$.

Toutes les administrations ont été réalisées par voie intra-gastrique, sous un volume constant (20 $ml.kg^{-1}$), à partir de solutions aqueuses.

Les animaux contrôles ont reçu un même volume d'eau distillée.

Les traitements sont réalisés 60 minutes avant le test.

2.)RESULTATS

2.1) Enceinte non anxiogène (actimètre)

Les valeurs moyennes du nombre de coupures de faisceaux sont rapportées sur le Tableau I.

L'activité motrice spontanée d'exploration (0-5 min) est relativement importante chez les souris témoins puisque l'on relève en moyenne 91 coupures des faisceaux lumineux. Cette activité décline ensuite en fonction du temps puisqu'entre 5 et 15 min (activité basale non anxiogène), c'est-à-dire dans un laps de temps deux fois plus important, le nombre de faisceaux infra-rouges interrompus n'est que de 128. Sur l'ensemble de la période d'observation (0-15 min), le nombre des faisceaux infra-rouges franchis a été de 219 (Tableau I).

L'administration préalable de $\alpha$-$\beta$-aspartate de magnésium n'entraîne pas de variation franche proportionnelle à la posologie de l'activité motrice des souris. On observe, seulement en présence de 700 $mg.kg^{-1}$ P.O., une augmentation statistiquement significative tant de l'activité exploratoire (0-5 min) que de l'activité basale non anxiogène (5-15 min) (Tableau I).

Compte tenu de ces résultats, il est donc illusoire de chercher à calculer une DA 50.

2.2) Enceinte anxiogène (cylindre de verre)

Les valeurs moyennes du nombre de redressements observés sont rapportées sur le Tableau II.

Les redressements qui traduisent l'exploration des souris contrôles placées dans le cylindre de verre sont maximum au cours de la première minute (15) puis déclinent progressivement au cours du temps : 8 au cours de la 2ème minute et 7 au cours de la 3ème. Plus les souris sont anxieuses, moins le nombre de redressements est important au cours de la première minute. Par contre, la miction et la défécation sont plus abondantes et traduisent l'aspect émotionnel du comportement "anxieux". Ce comportement s'observe chez 17 souris sur 20, soit 85 p. 100 dans la série contrôle.

L'administration préalable de α-β-aspartate de magnésium aux doses de 700 et 1400 mg.kg-l, augmente significativement le nombre de redressements, en particulier au cours de la 2ème minute suivant l'introduction dans le cylindre (tableau 4). Ceci va de pair, à la dose de 700 $mg.kg^{-1}$ avec une diminution très importante du nombre des souris présentant une défécation et une miction émotionnelles (27 %). Ce pourcentage est également plus faible que chez les témoins aux deux doses les plus faibles : 175 $mg.kg^{-1}$ (40 %) et 350 $mg.kg^{-1}$ (53 %) mais est paradoxalement plus élevé à celle de 1400 $mg.kg^{-1}$ (73 %).

La DA 50 calculée à partir de ces pourcentages est égale à : 577 $mg.kg^{-1}$ avec des limites de confiance au niveau de probabilité 5 p. 100 égales à :

| | |
|---|---|
| limite inférieure | 160 $mg.kg^{-1}$ |
| limite supérieure | 2082 $mg.kg^{-1}$ |

2.3) Tonus musculaire

Cinq des 20 souris témoins chutent de la tige tournante au cours des 3 minutes d'observation (25 %). Après ingestion de α-β-aspartate de magnésium, ce pourcentage est plus faible (13 %) à dose faible (175 $mg.kg^{-1}$) mais franchement plus élevé (de 53 à 60 %) aux 3 autres doses.

La DA 50 calculée à partir de ces pourcentages, comme décrit par J.T. LITCHFIELD et F. WILCOXON (J. Pharm. Exp. Ther. 1949, 96 : 99-113) est égale à 612 $mg.kg^{-1}$ avec des limites de confiance au niveau de probabilité 5 p. 100 égales à :

| | |
|---|---|
| limite inférieure | 343 $mg.kg^{-1}$ |
| limite supérieure | 1091 $mg.kg^{-1}$ |

CONCLUSIONS

Au terme de cette étude, on peut dire que l'α-β-aspartate de magnésium exerce une activité anti-émotionnelle avec une DA 50 égale à 577 $mg.kg^{-1}$ P.O., qu'elle s'accompagne d'un maintien, voire d'un accroissement de l'activité exploratoire dans ces enceintes anxiogène et non anxiogène, et d'une réduction du tonus musculaire (DA 50 = 612 $mg.kg^{-1}$).

On peut donc conclure que l'α-β-aspartate de magnésium possède des propriétés tranquillisantes mineures sans effet sédatif, mais s'accompagnant d'une légère hypotonie musculaire, pour une dose d'environ 600 $mg.kg^{-1}$ qui représente environ le 1/10 de la DL 50.

**EXEMPLE 3 : Effet de l'α-β-aspartate de magnésium en administration réitérée, sur le comportement exploratoire et la coordination motrice chez la souris**

PROTOCOLE EXPERIMENTAL

Il est identique à celui décrit dans l'exemple précédent.

Cent vingt cinq souris ont été réparties de la façon suivante :

| Séries d'animaux | Doses mg.$kg^{-1}$ P.O | Nombre |
|---|---|---|
| Contrôle | 0 | 20 |
| $\alpha$-$\beta$-aspartate de magnésium | 10,95<br>21,9<br>43,8<br>87,5<br>175<br>350<br>700 | 15<br>15<br>15<br>15<br>15<br>15<br>15 |

Les doses ont été calculées selon une progression géométrique de base 700 (correspondant au 1/10 de la DL 50) et de raison 2, soit respectivement 10,95 ; 21,9 ; 43,8 ; 87,5 ; 175 ; 350 ; 700 mg/kg. Toutes les administrations ont été réalisées par voie intragastrique, sous un volume constant (20 ml/kg), à partir de solutions aqueuses pendant 15 jours consécutifs. Les animaux contrôles ont reçu un même volume d'eau distillée. Les derniers traitements ont été réalisés 60 minutes avant le test.

2) RESULTATS

2.1) Enceinte non anxiogène (actimètre)

Les valeurs moyennes sont rapportées sur le tableau III. L'activité motrice spontanée d'exploration (0-5 min) est relativement importante chez les souris témoins puisque l'on relève en moyenne 88 coupures des faisceaux lumineux. Cette activité décline ensuite en fonction du temps puisqu'entre 5 et 15 min (activité basale non anxiogène), c'est-à-dire dans un laps de temps deux fois plus important, le nombre de faisceaux infra rouges interrompus n'est que de 112. Sur l'ensemble de la période d'observation (0-15 min), le nombre des faisceaux infra-rouges franchis a été de 200.

L'administration réitérée (13 jours) d'$\alpha$-$\beta$-aspartate de magnésium entraîne, comparativement aux contrôles, une élévation significative tant de l'activité exploratoire (0-5 min) que de l'activité basale non anxiogène (5-15 mn). Cet effet, particulièrement net aux doses de 43,8 et 350 mg/kg, semble être propre à l'administration d'$\alpha$-$\beta$-aspartate de magnésium mais non proportionnel à la dose. La durée de l'imprégnation (13 jours) avant le test peut expliquer cette absence de proportionalité dose/effet. Le calcul d'une DA 50 est dans ces conditions impossible.

2.2) Enceinte anxiogène (cylindre de verre)

Les valeurs moyennes sont rapportées dans le tableau IV.

Les redressements qui traduisent l'exploration des souris contrôles placées dans le cylindre de verre sont maximum au cours de la première minute (13) puis déclinent progressivement au cours du temps : 10 au cours de la 2ème minute et 7 au cours de la 3ème. Plus les souris sont anxieuses, moins le nombre de redressements est important au cours de la première minute. Par contre, la miction et la défécation sont plus abondantes et traduisent l'aspect émotionnel du comportement "anxieux". Ce comportement s'observe chez 20 souris sur 25, soit 80 p. 100 dans la série contrôle.

L'administration réitérée d'$\alpha$-$\beta$-aspartate de magnésium provoque chez la souris une tendance à l'augmentation du nombre de redressements traduisant une réduction de l'anxiété . Cet effet, cependant, n'est pas proportionnel à la dose puisqu 'on l'observe a différents moments de durée de 3 minutes du test et pour des doses aussi différentes que 700 ou 10,95 mg/kg. Le comportement mictionnel et défécatoire est en revanche plus révélateur de l'effet du traitement. En effet si 20 % des animaux contrôle ne semblent pas présenter de comportement anxiogène, l'administration d'$\alpha$-$\beta$-aspartate de magnésium élève cette valeur à 40 % pour la dose de 10,95 et 21,9 mg/kg, à 60 % pour la dose de 43,8 mg/kg, à 66,7 % pour la dose de 87,5 mg/kg et à 73,3 % pour la dose de 175 mg/kg. Les deux doses de 350 et 700 mg/kg entraînent un pourcentage respectif de 60 et 66,7 %, soit un léger infléchissement de l'effet. Ces résultats, en revanche, permettent le calcul d'une DA 50, basé sur le nombre de sujets ne présentant pas de comportement anxieux. Ce calcul donne pour l'$\alpha$-$\beta$-aspartate de magnésium la dose de 27,81 mg/kg (administration réitérée sur 13 jours) avec comme limite supérieure 68,66 mg/kg et comme limite inférieure 11,26 mg/kg.

2.3) Tonus musculaire

La recherche d'un effet relaxant musculaire de l'α-β-aspartate de magnésium est rapportée dans le tableau V.

Dans le groupe contrôle, 5 souris sur 25 chutent de la tige tournante au cours des 3 minutes d'observation (20 %). L'administration réitérée d'α-β-aspartate de magnésium provoque une légère élévation de ce pourcentage sans que l'on puisse détecter une relation dose/effet. On note un effet relaxant musculaire croissant de la dose de 10,95 mg/kg (26,6 %) à la dose de 43,8 mg/kg (46,6 %) puis un plateau lorsque les doses sont augmentées.

Le calcul d'une DA 50 n'est, sur ce test, pas possible du fait de l'absence notable d'effet/dose.

CONCLUSION

Dans l'exemple précédent a été mise en évidence une activité tranquillisante mineure ou anti-émotionnelle de l'α-β-aspartate de magnésium en administration unique pour des doses comprises entre 500 et 600 mg/kg. La réitération de l'administration de l'α-β-aspartate de magnésium pendant 13 jours a permis non pas d'exacerber cette activité anxiolytique, mais de réduire considérablement les doses qui dans le cadre de l'enceinte anxiogène sont de l'ordre de 30 mg/kg (DA 50 = 27,8 mg/kg).

Au terme de cette étude on peut conclure à la présence d'une activité tranquillisante mineure de l'α-β-aspartate de magnésium. Cette activité est étroitement conditionnée, aux doses utilisées, par le caractère réitéré de l'administration.

**EXEMPLE 4 - Effet anxiolytique mineur de l'α-β-aspartate de magnésium : mise en évidence par le test des 4 plaques**

En 1968, J.R. BOISSIER et al. (EUR. J. Pharmacol. 4 : 145) ont proposé une méthode rapide de screening pour des tranquillisants mineurs : le test dit des 4 plaques qui est basé sur un apprentissage suppressif . Ce test classique permet de mettre en évidence l'effet du α-β-aspartate de magnésium sur le comportement anxieux de la souris.

1) PROTOCOLE EXPERIMENTAL

Le test est pratiqué chez des souris NMRI-HAN soumises à une diète hydrique préalable de 18 heures et réparties en séries de 15.

L'appareillage utilisé consiste en une boite en plexiglas dont le sol est constitué de 4 plaques métalliques séparées les unes des autres. Un circuit d'alimentation permet d'établir une différence de potentiel entre deux plaques adjacentes grâce à un contacteur. Le test est pratiqué en plaçant doucement une souris sur une des plaques du fond de l'appareil ; les plaques sont électrifiées chaque fois que la souris passe de l'une à l'autre, de telle sorte qu'elle reçoit alors un choc électrique au niveau des pattes. Les paramètres du choc sont les suivants : durée de l'impulsion électrique égale à 0,5 seconde ; intensité égale à 0,6 milliampères. Ce choc induit chez les animaux une réaction motrice évidente de fuite, la souris traversant souvent 2 à 3 plaques successivement. Les conditions expérimentales adoptées ont été les suivantes : exploration libre pendant 15 secondes, traversée punie pendant la minute suivante, les résultats étant exprimés en nombre de passages "punis" pendant cette minute.

Soixante souris ont été réparties de la façon suivante :

| Séries d'animaux | Doses $mg.kg^{-1}$ P.O | Nombre |
|---|---|---|
| Contrôle | 0 | 15 |
| α-β-aspartate de magnésium | 300<br>600<br>1200 | 15<br>15<br>15 |

Les administrations ont été réalisées par voie intra-gastrique, à partir de solutions aqueuses, sous un volume constant de 20 $ml.kg^{-1}$, 60 minutes avant le début du test. Les animaux contrôles ont reçu un même volume d'eau distillée.

2) RESULTATS

Les données moyennes sont rapportées sur le tableau VI.

Après 15 minutes d'exploration, le nombre de passages d'une plaques à l'autre réalisée en une minute par une souris naïve placée dans cette situation expérimentale mais ne subissant aucune punition est de l'ordre de 15. Ceci traduit une période d'intense activité exploratoire.

Lorsque chaque passage est "puni" par un choc électrique, on constate l'établissement rapide d'un conditionnement. L'apprentissage suppressif ainsi mis en oeuvre se traduit par une réduction considérable de la motilité avec un nombre de passages qui chute chez les souris témoins aux environs de 6, par estimation du risque encouru.

Administré 60 minutes avant la réalisation du test, à la dose de 300 $mg.kg^{-1}$, l'$\alpha$-$\beta$-aspartate de magnésium n'accroît pas significativement le nombre de passages "punis". Par contre, aux deux doses de 600 et 1200 $mg.kg^{-1}$, ce nombre est accru significativement, de façon maximum à la dose de 600 $mg.kg^{-1}$. La diminution de la performance à la dose de 1200 $mg.kg^{-1}$ pourrait être en rapport avec une diminution du tonus musculaire (DA 50 = 612 $mg.kg^{-1}$).

Cet exemple confirme que l'$\alpha$-$\beta$-aspartate de magnésium possède des propriétés tranquillisantes mineures, avec une dose optimum égale à 600 $mg.kg^{-1}$.

B) MISE EN EVIDENCE D'UNE POTENTIALISATION DE L'EFFET ANXIOLYTIQUE DES BENZODIAZEPINES PAR L'$\alpha$-$\beta$-ASPARTATE DE MAGNESIUM

**EXEMPLE 5 : Effet anxiolytique d'une association $\alpha$-$\beta$-aspartate de magnésium/diazepam en prise unique : mise en évidence par le test des 4 plaques chez la souris**

1) PROTOCOLE EXPERIMENTAL

Ce test est pratiqué selon le protocole décrit à l'exemple 4.

Cent cinquante souris ont été réparties de la façon suivante :

| Séries d'animaux | Doses $mg.kg^{-1}$ P.O | Nombre |
|---|---|---|
| Contrôle | 0 | 30 |
| Diazépam | 0,25<br>0,5<br>1<br>2 | 15<br>15<br>15<br>15 |
| Diazépam + $\alpha$-$\beta$-aspartate de magnésium 600$mg.kg^{-1}$ | 0,125<br>0,25<br>0,5<br>1 | 15<br>15<br>15<br>15 |

Les administrations ont été réalisées par voie intra-gastrique, à partir de solutions aqueuses, sous un volume constant de 20 $ml.kg^{-1}$ par produit 60 minutes avant le début du test. Les animaux contrôles ont reçu un même volume d'eau distillée.

Dans un premier temps a été calculée la DA 50 (dose anxiolytique 50) du Diazépam (Valium® 10 mg - Roche, ampoule 2ml) seul, administré à des doses suivant une progression géométrique de base 0,25 et de raison 2, soit respectivement 0,25, 0,5 l et 2 $mg.kg^{-1}$.

Dans un deuxième temps a été recherchée la DA 50 du Diazépam en association avec l'$\alpha$-$\beta$-aspartate de magnésium à la dose de 600 mg (soit une progressiongéométrique de base 0,125 et de raison 2, soit respectivement 0,125, 0,25, 0,5 et 1 $mg.kg^{-1}$).

La DA 50 est déterminée à partir du pourcentage d'animaux tranquillisés. Est considéré comme tranquillisé tout animal dont le nombre de passages punis est égal ou supérieur à 9, c'est-à-dire à une valeur représentant une augmentation d'au moins 50 % par rapport au score moyen d'animaux non traités. Le calcul de la DA 50 a été réalisé selon la méthode graphique de J.T. LITCHFIELD et F. WILCOXON (J. Pharm. Exp. Ther. 1949, 96 : 99-113).

2) RESULTATS

Les données moyennes sont rapportées sur le tableau VII.

La dose anxiolytique 50 du Diazépam seul, dans ces conditions expérimentales est de 0,76 $mg.kg^{-1}$. Lors de l'association au Diazépam de la dose de 600 mg.kg-l de α-β-aspartate de magnésium, on constate une réduction de la DA 50 qui, dès lors, est égale à 0,32 $mg.kg^{-1}$.

Toutefois la comparaison des DA 50 des deux groupes ne met pas en évidence de différence statistiquement significative bien que le rapport d'activité soit de 2,38

CONCLUSION

Bien que l'on observe une diminution de la dose anxiolytique 50 du diazépam lorsqu'il est associé à la dose de 600 mg de α-β-aspartate de magnésium, cette différence n'atteint pas le niveau statistique de $p \leqq 0,05$. Sur la base de cette observation on peut se demander si le bénéfice de cette association est optimal dans le cadre d'une administration unique de 600 mg de α-β-aspartate de magnésium et si l'effet adjuvant ne serait pas plus marqué en administrant l'α-β-aspartate de magnésium de façon répétée à une dose moindre.

**EXEMPLE 6 - Potentialisation de l'effet anxiolytique du diazépam par des administrations réitérées d'α-β-aspartate de magnésium : Mise en évidence par le test des 4 plaques chez la souris**

1) PROTOCOLE EXPERIMENTAL

Le test a été réalisé comme décrit à l'exemple précédent.

Cent cinquante souris ont été réparties de la façon suivante :

| Séries d'animaux | Doses $mg.kg^{-1}$ P.O | Nombre |
|---|---|---|
| Contrôle | 0 | 30 |
| Diazépam | 0,25<br>0,5<br>1<br>2 | 15<br>15<br>15<br>15 |
| Diazépam + α-β-aspartate de magnésium 28$mg.kg^{-1}$x13 | 0,125<br>0,25<br>0,5<br>1 | 15<br>15<br>15<br>15 |

Toutes les administrations ont été réalisées par voie intra-gastrique à partir de solutions aqueuses, sous un volume constant de 20 $ml.kg^{-1}$ (eau distillée pour le groupe contrôle). L'α-β-aspartate de magnésium a été administré quotidiennement durant 13 jours consécutifs à la dose de 28 $mg.kg^{-1}$ P.O.(DA 50 de l'α-β-aspartate de magnésium déterminée dans une enceinte anxiogène, Cf exemple 3). La veille du test, les souris sont soumises à une diète hydrique de 18 heures. La dernière ingestion est pratiquée une heure avant l'étude, concomitamment avec le diazépam (Valium® 10 mg - Roche, ampoules de 2 ml).

2) RESULTATS

Le nombre moyen de passages d'une plaque à l'autre sont rapportées sur le tableau VIII (diazépam/α-β-aspartate de magnésium)

Les DA 50 du diazépam seul ou associé à l'α-β-aspartate de magnésium ont été calculées à partir du pourcentage de souris tranquillisées

Qu'il s'agisse du diazépam seul ou associé à l'α-β-aspartate de magnésium, on observe par rapport à la série témoin, une augmentation statistiquement significative du nombre de "passage punis" entre deux plaques adjacentes.

Les DA 50 ont été calculées, comme décrit précédemment.

On constate ainsi que la dose anxiolytique 50 du diazépam seul est égale à 0,76 $mg.kg^{-1}$. Mais lorsque l'administration du diazépam est précédée de 13 ingestions quotidiennes consécutives d'α-β-aspartate de

magnésium, la DA 50 du diazépam est réduite à 0,28 $mg.kg^{-1}$.

La comparaison de ces DA 50 fait apparaître que l'administration répétée d'aspartate de magnésium réduit de façon statistiquement significative ($p \leqq 0,05$) la dose anxiolytique 50 du diazépam, le rapport d'activité étant égal à 2,71.

CONCLUSION

Au terme de cette étude, il a été clairement démontré que l'administration préalable et réitérée (13 jours) d'$\alpha$-$\beta$-aspartate de magnésium potentialise fortement l'effet anxiolytique du diazépam. En effet, dans le test classique dit des 4 plaques, la DA 50 du diazépam a été réduite d'environ 2/3 : 0,28 $mg.kg^{-1}$ au lieu de 0,76 $mg.kg^{-1}$ en l'absence de prétraitement.

**EXEMPLE 7 : Potentialisation de l'effet anxiolytique du diazépam par l'$\alpha$-$\beta$-aspartate de magnésium chez le singe : mise en évidence par électrocortigraphie quantifiée**

Cette étude a pour but de caractériser l'effet d'une dose liminaire de diazépam grâce à un EEG quantifié et de rechercher une potentialisation de cet effet lorsque la même dose de diazépam est ingérée après administration réitérée préalable d'$\alpha$-$\beta$-aspartate de magnésium.

L'application de l'électroencéphalographie en psychopharmacologie date de la découverte de la chlorpromazine. Initialement l'analyse de l'EEG reposait sur une interprétation visuelle du tracé. Aujourd'hui, la sophistication des méthodes d'analyse grâce à l'ordinateur a permis de montrer par exemple que les drogues psychotropes ont, pour une dose donnée, un "profil" caractéristique propre, semblable à une empreinte, que l'EEG quantifié permet d'objectiver. Ainsi, les benzodiazépines engendrent une recrudescence des rythmes les plus rapides (bêta) avec une fréquence dominante spécifique à chaque substance alors qu'au contraire un neuroleptique va provoque un glissement vers des fréquences plus lentes

1) PROTOCOLE EXPERIMENTAL

L'étude a été réalisée chez sept singes cynomolgus (Macaca fascicularis), de sexe mâle et femelle, pesant entre 2 et 5 kg.

1.1) Mise en place des électrodes et enregistrement de l'ECG.

Un mois avant le début de l'étude, sous anesthésie générale (kétamine + xylazine) huit électrodes (boules d'argent) ont été implantées dans la table osseuse, au niveau des aires frontale, centrale, pariétale et occipitale droites et gauche.
L'électrocorticogramme a été enregistré sur un électroencéphalographe REEGA mini-huit (Alvar Electronic) en dérivations monopolaires par rapport à une référence commune.

Les enregistrements ont été réalisés sur des animaux habitués au siège à contention, en position assise, dans une pièce calme mais non insonorisée, en ambiance lumineuse tamisée.

1.2) Analyse spectrale de l'ECG

Les huit dérivations EEG ont été analysées simultanément par un Neuromapper 1620 (Neuroscience) qui extrait du signal les transformées de Fourier ("Fast Fourier Transform" : FFT). En fait, 90 échantillons de 2 secondes, soit 3 minutes de tracé exempt d'artefact, ont été analysés. Les données recueillies correspondent à l'amplitude ($\mu V$) de chacune des 5 bandes de fréquence EEG classiques selon les bornes suivantes : delta de 0 à 4 Hz ; thêta de 7 à 8 Hz ; alpha de 8 à 13 Hz, $bêta_1$ de 13 à 20 Hz ; $bêta_2$ de 20 à 30 Hz.

Pour chacune de ces bandes ont ensuite été calculées :

- Les puissances absolues en $\mu V^2$
- les puissances relatives (%), c'est-à-dire la puissance de chacune des bandes de fréquence rapportée à la puissance totale du spectre (power)
- la "SEF 95 % (Spectral Edge Frequency 95 %) qui est la fréquence en deçà de laquelle se situent 95 % de la puissance totale du spectre,
- le rapport entre fréquences rapides et fréquences lentes : alpha + bêta/delta + thêta ($\alpha + \beta/\delta + \theta$)

1.3) Les traitements

L'α-β-aspartate de magnésium a été administré durant deux semaines consécutives par voie orale (tubage naso-oesophagien), à raison de deux ingestions quotidiennes, à la dose de 50 $mg.kg^{-1}$, sous un volume de 2 $ml.kg^{-1}$ (les Samedis et Dimanches, la dose journalière, soit 100 $mg.kg^{-1}$ a été administrée en une fois). La dernière ingestion a précédé de 1 heure la seconde administration de diazépam.

Le diazépam (ampoules injectables - Valium® 10 - Roche) a été administré par voie orale, en solution aqueuse, sous un volume de 10 ml/singe, à des doses comprises entre 0,5 $mg.kg^{-1}$ et 4 $mg.kg^{-1}$ (doses différentes du fait d'une sensibilité individuelle propre à chaque sujet).

2)RESULTATS

Sur les figures 3 et 4 est illustrée l'évolution en fonction du temps de la SEF 95 % et du rapport $\alpha + \beta/\delta + \theta$.

Une analyse statistique a été réalisée selon le test t de Fisher-Student pour séries appariées (2 bornes) afin de comparer pour chaque paramètre les valeurs obtenues à chaque temps expérimental suivant l'ingestion de diazépam à la valeur ***ante*** obtenue, soit avant toute administration médicamenteuse, soit au terme des deux semaines d' imprégnation par l'α-β-aspartate de magnésium. Les résultats observés sont statistiquement significatifs (2 bornes) au niveau de probabilité 10 p. 100 (le seuil de probabilité statistique choisi peut paraître élevé ; il est en rapport avec le faible nombre de sujets : n = 7). L'ensemble de l'analyse statistique a été obtenu grâce au programme statistique SPSS/$PC^+$ (SPSS Inc., Chicago)

2.1) Effet de l'administration de Diazépam seul

L'administration de doses liminaires de diazépam, doses comprises entre 0,5 et 4 $mg.kg^{-1}$ P.O., a conduit à observer une recrudescence des rythmes rapides de fréquence bêta chez le singe.

Les premiers signes visuellement décelables de l'effet du diazépam surviennent aux environs de 6 à 8 minutes après ingestion. Il se caractérisent par des bouffées sporadiques d'ondes pointues rapides, sur un tracé dont l'amplitude s'accroît légèrement. L'acmé se situe en général aux alentours de 45 minutes à une heure, mais l'action perdure (plus ou moins marquée) jusqu'à la 5ème heure.

L'analyse spectrale de l'EEG révèle que l'effet du diazépam se caractérise, comme chez l'homme, par un enrichissement en rythmes rapides, en particulier dans la bande b, mais aussi parfois dans la bande alpha. Cependant, du fait du nombre restreint de sujets (n = 7), ces variations s'assortissent d'une grande variabilité. Parallèlement à la recrudescence des rythmes de fréquence rapide, les ondes les plus lentes : delta et thêta, se raréfient notablement.

L'ensemble de ces variations se résume dans une augmentation durable des valeurs du rapport $\alpha + \beta/\delta + \theta$ (figure 4).

Enfin, l'augmentation procentuelle des fréquences rapides provoque un glissement vers la droite du spectre de fréquence EEG, que traduit une augmentation de la "SEF 95 %" (figure 3).

2.2) Effet de l'association diazepam/α-β-aspartate de magnésium

L'administration réitérée d'α-β-aspartate de magnésium à raison de deux ingestions quotidiennes, durant deux semaines consécutives, à la dose de 50 $mg.kg^{-1}$, entraîne en soi des modifications de l'EEG de repos. Ces variations sont discrètes en moyenne car elles intéressent en fait 4 sujets sur 7. Elles portent essentiellement sur une recrudescence de rythmes rapides, la puissance relatives par exemple de la bande $\beta$ étant accrue de 4,4, 3,6, 6,2 et 13 % respectivement chez ces 4 sujets. La variation des autres bandes de fréquence est plus aléatoire, sauf celle des ondes delta qui est réduite de 2 à 10 % chez ces mêmes 4 singes. Chez les 3 autres, elle est soit inchangée, soit accrue de 5 % et 8 % respectivement. Ces variations ne sont pas suffisamment amples pour que les valeurs du rapport rythmes rapides/fréquences lentes soient significativement accrues (figure 4) ; par contre la "SEF 95%" est augmentée de 0,5 Hz (figure 3). Bien que ces modifications soient en intensité nettement moindres, elles sont de même nature que celles enregistrées avec le diazépam, en particulier la recrudescence du $\beta$ au détriment des rythmes lents.

L'ingestion de diazépam réalisée une heure après la dernière administration d'α-β-aspartate de magnésium, donne naissance à des modifications EEG semblables en nature à celles observées lors de l'ingestion du diazépam seul, mais globalement plus intenses. Par exemple, la proportion relative de la bande $\beta_2$ s'accroit de 23 % initialement (avant diazépam) jusqu'à 30 % en moyenne deux heures après l'ingestion de diazépam. Rappelons que lors du premier test, cette proportion relative variait de 20 %

(valeur de référence) jusqu'à 27 % à l'acmé de l'effet du diazépam obtenue alors une heure après l'ingestion.

D'une manière générale, il apparaît qu'après imprégnation par l'$\alpha$-$\beta$-aspartate de magnésium, l'effet tranquillisant du diazépam tel qu'il s'exprime au niveau EEG, est plus intense mais surtout plus durable.

Ceci est parfaitement illustré sur la figure 3 qui retrace l'évolution de la "SEF 95 %". Lors du premier test, cette fréquence s'accroît progressivement jusqu'à atteindre un maximum à la 30ème minute et demeure peu ou prou en plateau jusqu'à une heure ; puis l'effet s'atténue légèrement à partir de 1 h 30 mais néanmoins perdure jusqu'à la 5ème heure. Lors du second test, l'"effet diazépam" est d'emblée plus marqué ; il n'atteint son acmé qu'à 1 h 30 - 2 h et ne et ne s'infléchit qu'à peine au cours des heures suivantes.

De la même manière l'ampleur de l'accroissement des valeurs du rapport $\alpha + \beta/\delta + \theta$ est remarquablement plus grande après traitement par l'$\alpha$-$\beta$-aspartate de magnésium et la durée d'effet également prolongée (figure 4)

## CONCLUSION

L'administration d'$\alpha$-$\beta$-aspartate de magnésium seul pendant deux semaines consécutives engendre, chez le singe vigile, des modifications EEG moindres mais de même nature que celles engendrées par le diazépam, c'est-à-dire une recrudescence des rythmes de fréquence rapide, essentiellement de la bande bêta, qui s'accompagne d'un appauvrissement en fréquences lentes.

Une administration de diazépam chez des sujets "imprégnés" d'$\alpha$-$\beta$-aspartate de magnésium génère dans 5 cas sur 7 un renforcement de l'effet préalablement observé en présence de diazépam seul ; non seulement cet effet est plus prononcé, mais il est aussi plus durable.

### Exemple 8 : Détermination de la DL 50 de l'$\alpha$-$\beta$-aspartate de magnésium chez la souris

La DL 50 de l'$\alpha$-$\beta$-aspartate de magnésium a été déterminée par voie intraveineuse et par voie intragastrique ; par voie intraveineuse elle est d'environ 238,4 $mg.kg^{-1}$ chez la souris male, et d'environ 230,5 $mg.kg^{-1}$ chez la souris femelle ; par voie intra-gastrique, elle est d'environ 7,1 $g.kg^{-1}$ chez la souris male, et d'environ 6,6 $g.kg^{-1}$ chez la souris femelle.

TABLEAU I - SCORE D'ACTIVITE MOTRICE CHEZ LA SOURIS DANS UN ACTIMETRE: α-β-ASPARTATE DE MAGNESIUM EN PRISE UNIQUE

| Activité motrice 0-5 minutes | | |
|---|---|---|
| Série | nombre d'animaux | moyenne |
| Contrôle | 20 | 91 |
| Mag:175 | 15 | 100 |
| Mag:350 | 15 | 96 |
| Mag:700 | 15 | 113 |
| Mag:1400 | 15 | 110 |
| **Activité motrice 5-15 minutes** | | |
| Série | nombre d'animaux | moyenne |
| Contrôle | 20 | 128 |
| Mag:175 | 15 | 72 |
| Mag:350 | 15 | 117 |
| Mag:700 | 15 | 145 |
| Mag:1400 | 15 | 104 |
| **Activité motrice 0-15 minutes** | | |
| Série | nombre d'animaux | moyenne |
| Contrôle | 20 | 219 |
| Mag:175 | 15 | 172 |
| Mag:350 | 15 | 213 |
| Mag:700 | 15 | 258 |
| Mag:1400 | 15 | 214 |

* Mag = α-β-Aspartate de magnésium

TABLEAU II - COMPORTEMENT EXPLORATOIRE CHEZ LA SOURIS DANS UNE ENCEINTE ANXIOGENE : α-β-ASPARTATE DE MAGNESIUM EN PRISE UNIQUE

| 1ère minute | | |
|---|---|---|
| Série | nombre d'animaux | moyenne |
| Contrôle | 20 | 15 |
| Mag:175 | 15 | 14 |
| Mag:350 | 15 | 15 |
| Mag:700 | 15 | 15 |
| Mag:1400 | 15 | 15 |
| 2ème minute | | |
| Série | nombre d'animaux | moyenne |
| Contrôle | 20 | 8 |
| Mag:175 | 15 | 8 |
| Mag:350 | 15 | 9 |
| Mag:700 | 15 | 10 |
| Mag:1400 | 15 | 11 |
| 3ème minute | | |
| Série | nombre d'animaux | moyenne |
| Contrôle | 20 | 7 |
| Mag: 175 | 15 | 7 |
| Mag: 350 | 15 | 6 |
| Mag: 700 | 15 | 7 |
| Mag:1400 | 15 | 8 |
| Total | | |
| Série | nombre d'animaux | moyenne |
| Contrôle | 20 | 30 |
| Mag: 175 | 15 | 29 |
| Mag: 350 | 15 | 30 |
| Mag: 700 | 15 | 32 |
| Mag:1400 | 15 | 34 |

TABLEAU III SCORE D'ACTIVITE MOTRICE CHEZ LA SOURIS DANS UN ACTIMETRE : α-β-ASPARTATE DE MAGNESIUM PRISE REITEREE

| Activité motrice 0-5 minutes | | |
|---|---|---|
| Série | nombre d'animaux | moyenne |
| Contrôle | 25 | 88 |
| Mag:10.95 | 15 | 98 |
| Mag:21.9 | 15 | 104 |
| Mag:43.8 | 15 | 104 |
| Mag:87.5 | 15 | 99 |
| Mag:175 | 15 | 106 |
| Mag:350 | 15 | 107 |
| Mag:700 | 15 | 104 |
| Activité motrice 5-15 minutes | | |
| Série | nombre d'animaux | moyenne |
| Contrôle | 25 | 112 |
| Mag:10.95 | 15 | 123 |
| Mag:21.9 | 15 | 133 |
| Mag:43.8 | 15 | 128 |
| Mag:87.5 | 15 | 96 |
| Mag:175 | 15 | 122 |
| Mag:350 | 15 | 141 |
| Mag:700 | 15 | 126 |
| Activité motrice 0-15 minutes | | |
| Série | nombre d'animaux | moyenne |
| Contrôle | 25 | 200 |
| Mag:10.95 | 15 | 221 |
| Mag:21.9 | 15 | 237 |
| Mag:43.8 | 15 | 232 |
| Mag:87.5 | 15 | 195 |
| Mag:175 | 15 | 228 |
| Mag:350 | 15 | 248 |
| Mag:700 | 15 | 230 |

TABLEAU IV

| 1ère minute | | |
|---|---|---|
| Série | nombre d'animaux | moyenne |
| Contrôle | 25 | 13 |
| Mag:10.95 | 15 | 16 |
| Mag:21.9 | 15 | 14 |
| Mag:43.8 | 15 | 13 |
| Mag:87.5 | 15 | 13 |
| Mag:175 | 15 | 12 |
| Mag:350 | 15 | 13 |
| Mag:700 | 15 | 14 |
| **2ème minute** | | |
| Série | nombre d'animaux | moyenne |
| Contrôle | 25 | 10 |
| Mag:10.95 | 15 | 11 |
| Mag:21.9 | 15 | 9 |
| Mag:43.8 | 15 | 9 |
| Mag:87.5 | 15 | 9 |
| Mag:175 | 15 | 9 |
| Mag:350 | 15 | 10 |
| Mag:700 | 15 | 10 |
| **3ème minute** | | |
| Série | nombre d'animaux | moyenne |
| Contrôle | 25 | 7 |
| Mag:10.95 | 15 | 8 |
| Mag:21.9 | 15 | 6 |
| Mag:43.8 | 15 | 8 |
| Mag:87.5 | 15 | 8 |
| Mag:175 | 15 | 8 |
| Mag:350 | 15 | 8 |
| Mag:700 | 15 | 9 |
| **Total** | | |
| Série | nombre d'animaux | moyenne |
| Contrôle | 25 | 30 |
| Mag:10.95 | 15 | 35 |
| Mag:21.9 | 15 | 29 |
| Mag:43.8 | 15 | 30 |
| Mag:87.5 | 15 | 30 |
| Mag:175 | 15 | 29 |
| Mag:350 | 15 | 31 |
| Mag:700 | 15 | 33 |

TABLEAU V

| RECHERCHE D'UN EFFET RELAXANT MUSCULAIRE DE L'$\alpha$-$\beta$-ASPARTATE DE MAGNESIUM | | | |
|---|---|---|---|
| Séries | Doses $mg.kg^{-1}$ P.O x13 | Nombre animaux | Animaux tombés (%) |
| Contrôle | - | 25 | 20,0 |
| $\alpha$-$\beta$ aspartate de magnésium | 10,95 | 25 | 26,6 |
| | 21,9 | 15 | 33,3 |
| | 43,8 | 15 | 46,6 |
| | 67,5 | 15 | 33,3 |
| | 175,0 | 15 | 46,6 |
| | 350,0 | 15 | 33,3 |
| | 700,0 | 15 | 33,3 |

TABLEAU VI - RECHERCHE D'UN EFFET ANXIOLYTIQUE CHEZ LA SOURIS : TEST DES 4 PLAQUES

| Nombre de passages | | |
|---|---|---|
| Série | nombre d'animaux | moyenne |
| Contrôle | 15 | 5,8 |
| Mag: 300 | 15 | 6,4 |
| Mag: 600 | 15 | 8,3 |
| Mag:1200 | 15 | 7,5 |

TABLEAU VII - RECHERCHE D'UN EFFET ANXIOLYTIQUE CHEZ LA SOURIS : TEST DES 4 PLAQUES - ETUDE DU VALIUM ET DE L'ASSOCIATION VALIUM-α-β-ASPARTATE DE MAGNESIUM

| Nombre de passages | | |
|---|---|---|
| Série | nombre d'animaux | moyenne |
| Contrôle | 15 | 5,5 |
| V (0,25) | 15 | 7,3 |
| V (0,5) | 15 | 7,7 |
| V (1) | 15 | 9,5 |
| V (2) | 15 | 10,6 |
| V+Mag:(0,125) | 15 | 7,5 |
| V+Mag:(0,25) | 15 | 8,0 |
| V+Mag:(0,5) | 15 | 9,3 |
| V+Mag:(1) | 15 | 9,6 |

V = Valium

TABLEAU VIII - RECHERCHE D'UN EFFET ANXIOLYTIQUE CHEZ LA SOURIS : TEST DES 4 PLAQUES - ETUDE DE L'ASSOCIATION VALIUM-α-β-ASPARTATE DE MAGNESIUM (28 mg/kgx13)

| Nombre de passages | | |
|---|---|---|
| Série | Nombre d'animaux | moyenne |
| Contrôle | 30 | 5,9 |
| V+Mag(0,125) | 15 | 7,5 |
| V+Mag(0,25) | 15 | 7,9 |
| V+Mag(0,5) | 15 | 10,5 |
| V+Mag(1,0) | 15 | 10,3 |

## Revendications

1. Sel d'acide aspartique et de magnésium, caractérisé en ce qu'il répond à la formule brute suivante :

   $C_4\ H_5\ O_4\ NMg,\ 2H_2O$.

2. Procédé de préparation d'un sel d'acide aspartique et de magnésium selon la revendication 1, lequel procédé est caractérisé en ce qu'il comprend une étape dans laquelle on procède à la déprotonation de l'acide aspartique en milieu hétérogène en présence d'hydroxyde de magnésium.

**3.** Procédé selon la revendication 2, caractérisé en ce que l'hydroxyde de magnésium est en excès molaire par rapport à l'acide aspartique.

**4.** Procédé selon l'une quelconque des revendications 2 ou 3, caractérisé en ce que le sel d'acide aspartique et de magnésium est récupéré à partir du mélange réactionnel par précipitation en présence d'acétone.

**5.** Utilisation d'un sel d'acide aspartique et de magnésium selon la revendication 1, pour l'obtention d'un médicament.

**6.** Composition pharmaceutique, caractérisée en ce qu'elle comprend en tant que principe actif un sel d'acide aspartique et de magnésium selon la revendication 1, seul ou en association avec un autre principe actif.

**7.** Composition pharmaceutique selon la revendication 6 caractérisé en ce qu'elle comprend en tant que principe actif un sel équimolaire d'acide aspartique et de magnésium en association avec un anxiolytique.

**8.** Composition pharmaceutique selon la revendication 7, caractérisée en ce que l'anxiolytique est une benzodiazépine.

**Claims**

**1.** Aspartic acid and magnesium salt, characterized in that it corresponds to the following empirical formula:

$C_4H_5O_4NMg \bullet 2H_2O$.

**2.** Process for the preparation of an aspartic acid and magnesium salt according to Claim 1, which process is characterized in that it comprises a step in which the aspartic acid is deprotonated in a heterogeneous medium in the presence of magnesium hydroxide.

**3.** Process according to Claim 2, characterized in that the magnesium hydroxide is in molar excess relative to the aspartic acid.

**4.** Process according to either of Claims 2 and 3, characterized in that the aspartic acid and magnesium salt is recovered from the reaction mixture by precipitation in the presence of acetone.

**5.** Use of an aspartic acid and magnesium salt according to Claim 1, for the production of a medicinal product.

**6.** Pharmaceutical composition, characterized in that it comprises, as active ingredient, an aspartic acid and magnesium salt according to Claim 1, alone or in combination with another active ingredient.

**7.** Pharmaceutical composition according to Claim 6, characterized in that it comprises, as active ingredient, an equimolar salt of aspartic acid and magnesium in combination with an anxiolytic agent.

**8.** Pharmaceutical composition according to Claim 7, characterized in that the anxiolytic agent is a benzodiazepine.

**Patentansprüche**

**1.** Magnesiumasparaginsäuresalz, dadurch **gekennzeichnet**, daß es der nachstehenden Bruttoformel entspricht:

$C_4H_5O_4NMg,\ 2H_2O$

2. Verfahren zur Herstellung eines Magnesiumasparaginsäuresalzes nach Anspruch 1, dadurch **gekennzeichnet**, daß man in einer Stufe die Asparaginsäure in einem heterogenen Medium in Gegenwart von Magnesiumhydroxid deprotoniert.

3. Verfahren nach Anspruch 2, dadurch **gekennzeichnet**, daß das Magnesiumhydroxid in einem molaren Überschuß, bezogen auf die Asparaginsäure, vorliegt.

4. Verfahren nach einem der Ansprüche 2 oder 3, dadurch **gekennzeichnet**, daß das Magnesiumasparaginsäuresalz aus dem Reaktionsgemisch durch Ausfällung in Gegenwart von Aceton isoliert wird.

5. Verwendung eines Magnesiumasparaginsäuresalzes nach Anspruch 1 zur Herstellung eines Medikaments.

6. Pharmazeutisches Präparat, dadurch **gekennzeichnet**, daß es als Wirkstoff ein Magnesiumasparaginsäuresalz nach Anspruch 1 allein oder zusammen mit einem anderen Wirkstoff umfaßt.

7. Pharmazeutisches Präparat nach Anspruch 6, dadurch **gekennzeichnet**, daß es als Wirkstoff ein equimolares Salz aus Magnesiumasparaginsäure zusammen mit einem Anxiolytikum umfaßt.

8. Pharmazeutisches Präparat nach Anspruch 7, dadurch **gekennzeichnet**, daß das Anxiolytikum ein Benzodiazepin ist.

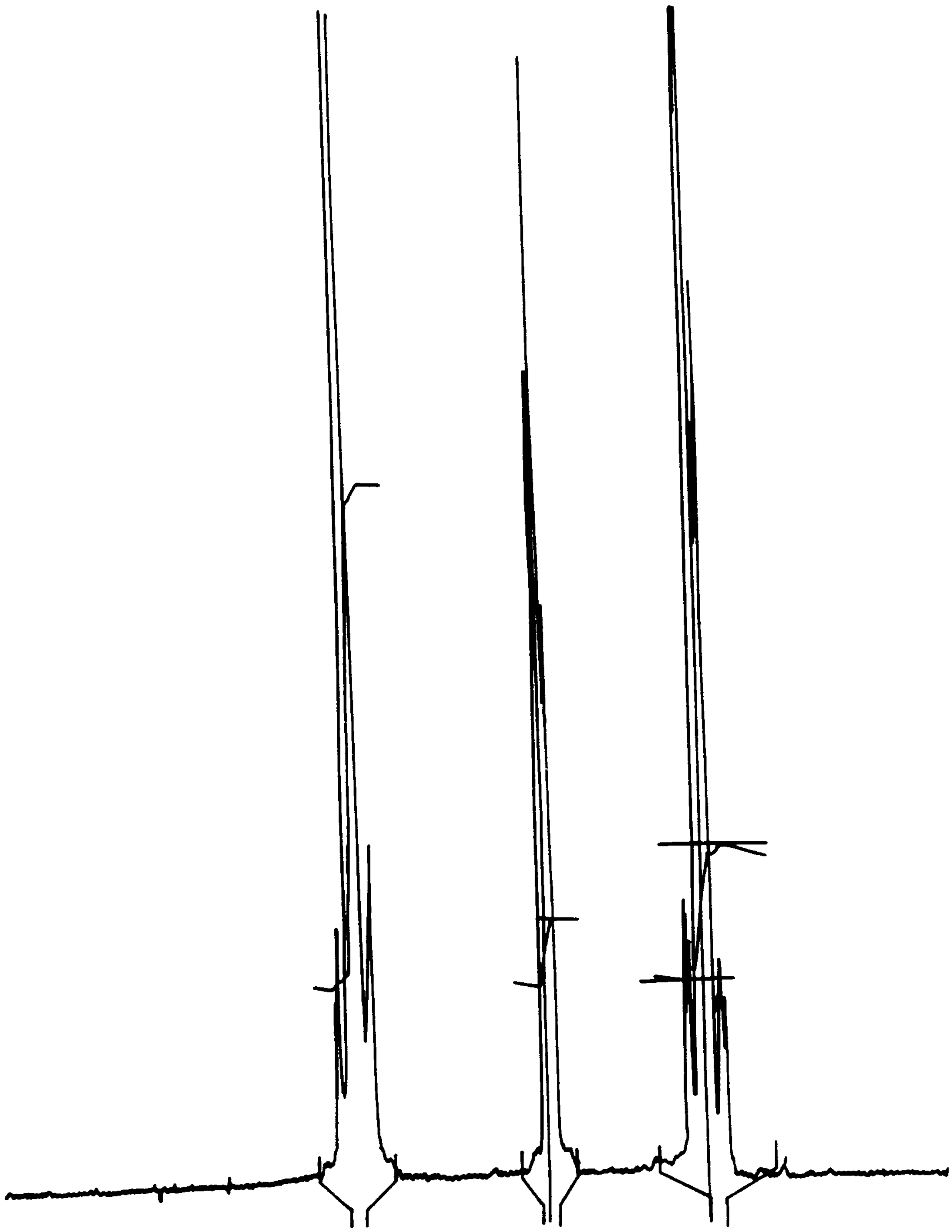

FIG. 1

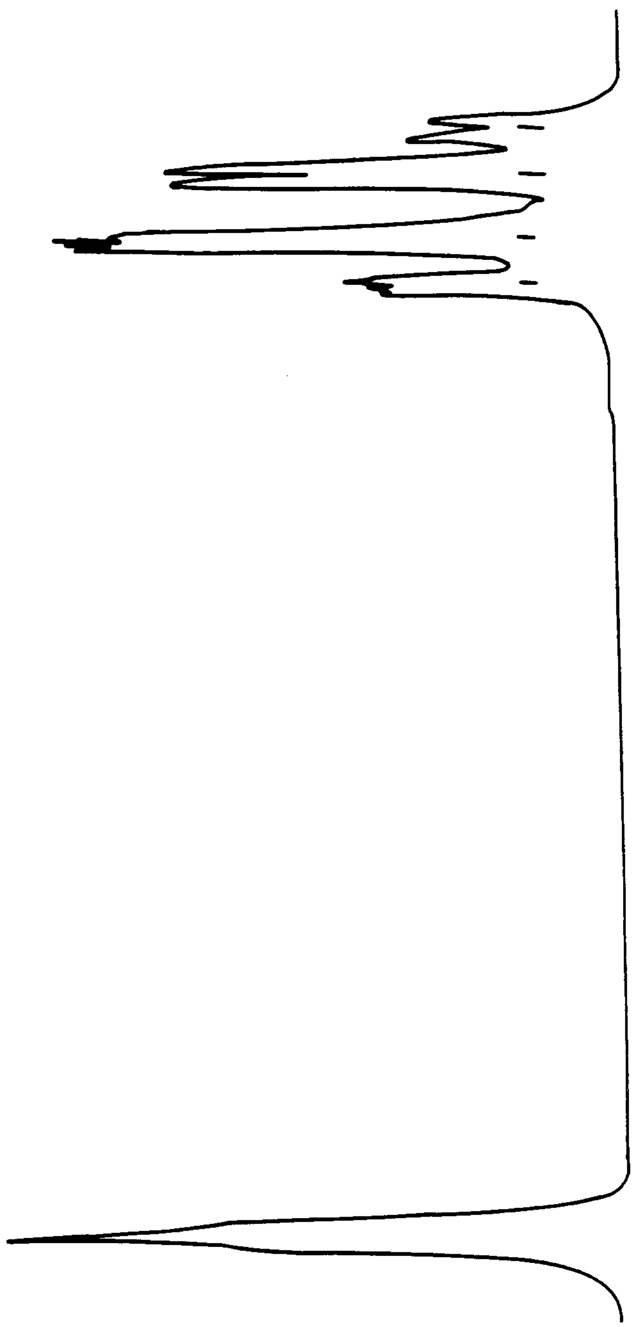

FIG. 2

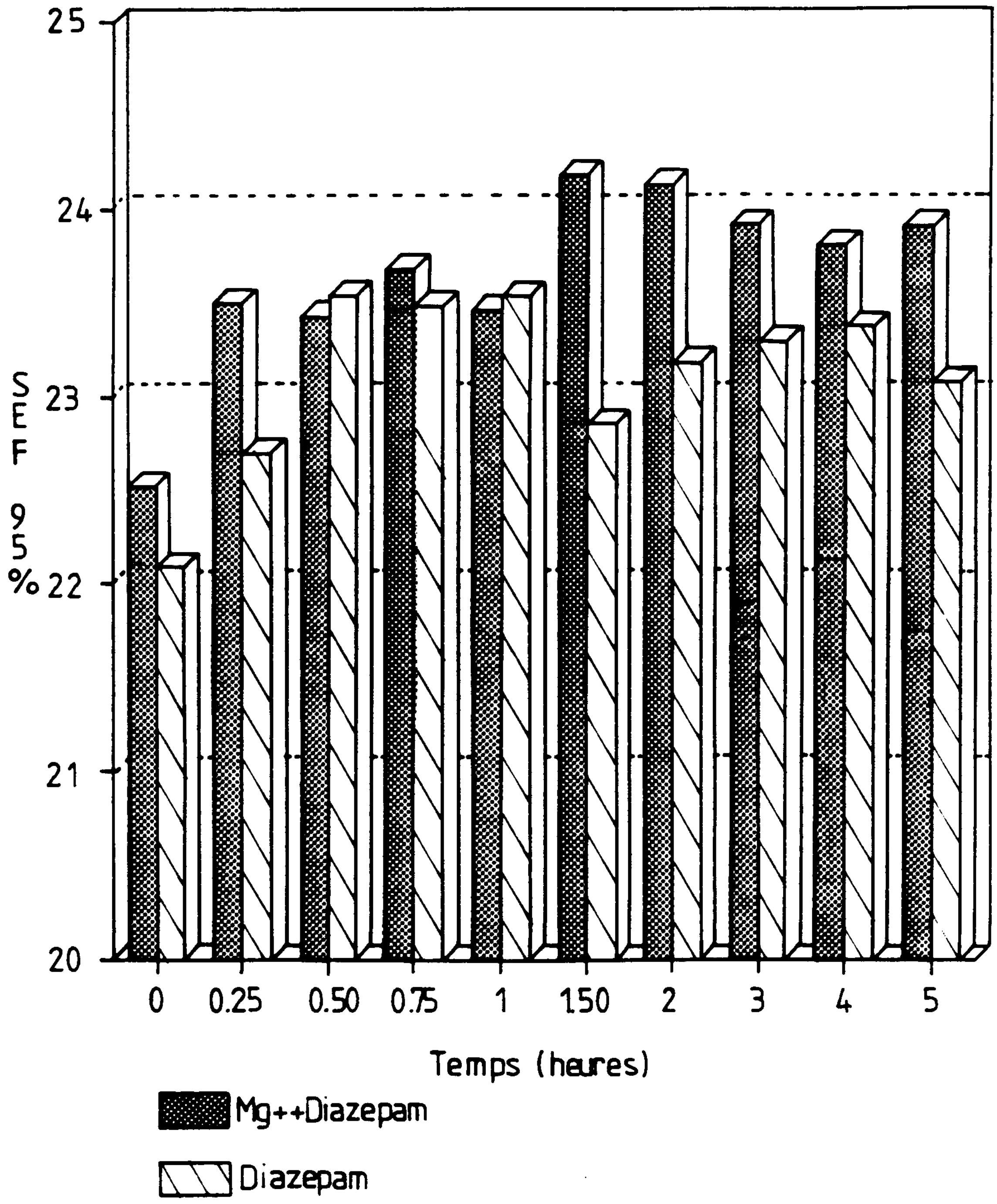
25
24
23
22
21
20
SEF 95%
0
0.25
0.50
0.75
1
1.50
2
3
4
5
Temps (heures)
Mg++Diazepam
Diazepam

FIG.3

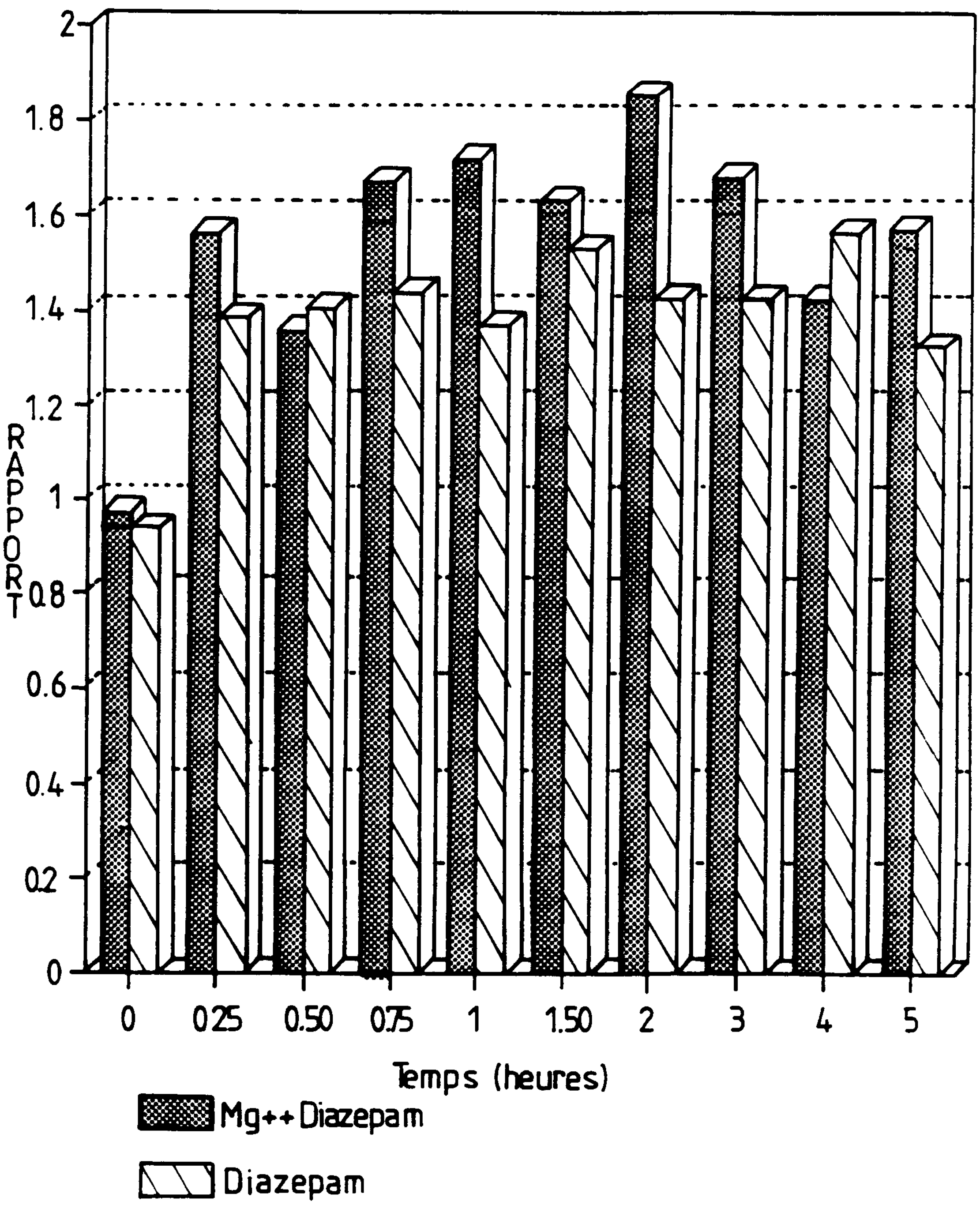
2
1.8
1.6
1.4
1.2
1
0.8
0.6
0.4
0.2
0
RAPPORT
0
0.25
0.50
0.75
1
1.50
2
3
4
5
Temps (heures)
Mg++Diazepam
Diazepam

FIG.4